# EUROPÄISCHE PATENTANMELDUNG

(11) **EP 2 165 718 A1**
(43) Veröffentlichungstag der Anmeldung: **24.03.2010**
(21) Anmeldenummer: 08164722.4
(22) Anmeldetag: 19.09.2008
(51) Int. Cl.: A61L 15/26, A61L 15/42, C08G 18/72, C08L 75/04, A61F 13/00

(54) **Wundauflage mit einer Polyurethan-Schaumschicht und einer Deckschicht aus thermoplastischem Polymer**

(71) Anmelder: Bayer MaterialScience AG, 51368 Leverkusen (DE)
(72) Erfinder: Die Erfindernennung liegt noch nicht vor

(57) **Zusammenfassung**

Die vorliegende Erfindung betrifft einen Schichtenverbund, geeignet als Wundauflage, umfassend eine Schaumschicht (10) sowie eine Deckschicht (20), wobei die Deckschicht (20) thermoplastisches Polymer, vorzugsweise Polyurethan, umfasst und zumindest teilweise direkt mit der Schaumschicht (10) verbunden ist und wobei die Schaumschicht (10) einen Polyurethanschaum umfasst, der erhalten wird, indem eine Zusammensetzung umfassend eine wässrige, anionisch hydrophilierte Polyurethandispersion (I) aufgeschäumt und getrocknet wird. Der Schaum kann mit Ethylenoxid/Propylenoxid-Blockcopolymeren stabilisiert werden. Die Erfindung betrifft weiterhin ein Verfahren zur Herstellung eines solchen Schichtenverbundes sowie dessen Verwendung beispielsweise als Wundauflage.

## Beschreibung

Die vorliegende Erfindung betrifft einen Schichtenverbund, geeignet als Wundauflage, umfassend eine Schaumschicht sowie eine Deckschicht, wobei die Deckschicht ein thermoplastisches Polymer umfasst und zumindest teilweise direkt mit der Schaumschicht verbunden ist. Sie betrifft weiterhin ein Verfahren zur Herstellung eines solchen Schichtenverbundes sowie dessen Verwendung beispielsweise als Wundauflage.

In der Versorgung von Wunden können Wundauflagen mit einer auf der Wunde liegenden Schaumschicht zum Einsatz kommen. Dieses hat sich als vorteilhaft erwiesen, da über die Aufnahmefähigkeit des Schaums für aus der Wunde austretende Feuchtigkeit ein der Heilung förderliches Klima in der Wunde erreicht werden kann. Solche Schäume an sich haben aber oft den Nachteil, dass sie verschmutzen, von Bakterien besiedelt oder durch die mechanischen Belastungen während des Tragens zerstört werden können.

Als Ausweg können Wundauflagen an ihrer Außenseite mit einer schützenden Deckfolie versehen werden. Diese Deckfolie kann für eine Keimdichtigkeit, für eine Dichtigkeit gegenüber aus der Wunde austretendem Exsudat bei gleichzeitiger Durchlässigkeit der Wundauflage für Wasserdampf sorgen. Bislang muss die Deckfolie mittels eines Klebstoffs mit der Schaumschicht verbunden werden.

Die Verwendung eines Klebstoffes bringt jedoch Nachteile mit sich. Das Aufbringen einer Klebstoffschicht bedeutet zusätzlichen Arbeits- und Materialaufwand und somit zusätzliche Kosten in der Fertigung einer Wundauflage. Weiterhin kann der Klebstoff die Durchlässigkeit gegenüber Wasserdampf ungünstig beeinflussen. Schließlich kann der Klebstoff auch Einfluss auf die thermische Verformbarkeit der Wundauflage nehmen.

WO 2007/115696, auf die in vollem Umfang Bezug genommen wird, offenbart ein Verfahren zur Herstellung von Polyurethan-Schäumen für die Wundbehandlung, bei welchem eine Zusammensetzung, enthaltend eine Polyurethan-Dispersion und spezielle Koagulantien, aufgeschäumt und getrocknet wird. Die Polyurethan-Dispersionen können beispielsweise erhalten werden, indem isocyanatfunktionelle Prepolymere aus organischen Polyisocyanaten und polymeren Polyolen mit zahlenmittleren Molekulargewichten von 400 g/mol bis 8000 g/mol und OH-Funktionalitäten von 1,5 bis 6 sowie gegebenenfalls mit hydroxyfunktionellen Verbindungen mit Molekulargewichten von 62 g/mol bis 399 g/mol und gegebenenfalls isocyanatreaktiven, anionischen oder potentiell anionischen und gegebenenfalls nichtionischen Hydrophilierungsmitteln hergestellt werden. Die freien NCO-Gruppen des Prepolymers werden dann ganz oder teilweise gegebenenfalls mit aminofunktionellen Verbindungen mit Molekulargewichten von 32 g/mol bis 400 g/mol sowie mit aminofunktionellen, anionischen oder potentiell anionischen Hydrophilierungsmitteln unter Kettenverlängerung umgesetzt. Die Prepolymere werden vor, während oder nach diesem Schritt in Wasser dispergiert. Gegebenenfalls enthaltene potentiell ionische Gruppen werden durch teilweise oder vollständige Umsetzung mit einem Neutralisationsmittel in die ionische Form überführt.

EP 0 485 657 offenbart eine Auflage für Wunden oder Hautgeschwüre. Die Auflage beinhaltet einen semipermeablen Polyurethanfilm und eine Mehrzahl von konzentrischen Polyethylenschaumringen. Das Aufbringen der Schaumringe auf einen semipermeablen dünnen Film ermöglicht die Beeinflussung der Feuchtigkeits-/Dampfdurchlässigkeits-Eigenschaften der Wundauflage. Diese können auf die Wundumgebung abgestimmt werden. Der semipermeable dünne Film ist mit der oberen Oberfläche eines Rings verbunden. Weiter wird offenbart, dass diese Verbindung mittels eines Klebstoffs erreicht wird.

DE 103 01 835 offenbart ein Pflaster mit einer bedruckten Wundauflage und einer transparenten Fixierfolie. Die Wundauflage kann individuell bedruckt und insbesondere unter Berücksichtigung der Konturen aufgedruckter Motive geformt werden. Die einseitig auf der wundzugewandten Seite klebende Fixierfolie überlappt die Wundauflage mindestens auf zwei Seiten und ist aus transparentem Material, wodurch die kosmetische Beeinträchtigung durch das Pflaster im wesentlichen auf die Größe der Wundauflage reduziert ist. Die überstehende klebende transparente Fixierfolie ermöglicht einen guten Halt, bei zusätzlicher Segmentierung auch in problematischen Hautregionen. Offenbart werden auch ein Verfahren zur Herstellung dieses Pflasters, seine Anwendung sowie Verfahren zur Anwendung. Die transparente Fixierfolie kann aus Polyurethan bestehen. Gemäß der Lehre dieser Veröffentlichung ist das transparente Fixierfolienmaterial auf der wundzugewandten Seite mit einem transparenten Klebstoff versehen, der einerseits für die Fixierung des Pflasters auf der Haut und andererseits für die Fixierung der Wundauflage auf der Fixierfolie sorgt.

Es besteht folglich der Bedarf an alternativen Wundauflagen. Insbesondere besteht der Bedarf an Wundauflagen, bei denen eine Schaumschicht und eine Deckschicht ohne Klebstoff in einer den Gebrauch nicht einschränkenden Festigkeit miteinander verbunden sind und welche sich thermisch verformen lassen.

Erfindungsgemäß vorgeschlagen wird daher ein Schichtenverbund, geeignet als Wundauflage, umfassend eine Schaumschicht sowie eine Deckschicht, wobei die Deckschicht ein thermoplastisches Polymer umfasst und zumindest teilweise direkt mit der Schaumschicht verbunden ist und wobei die Schaumschicht einen Polyurethanschaum umfasst, der erhalten wird, indem eine Zusammensetzung umfassend eine wässrige, anionisch hydrophilierte Polyurethan-dispersion (I) aufgeschäumt und getrocknet wird.

Es ist vorgesehen, dass die Schaumschicht einen Schaum umfasst, welcher aus einer aufgeschäumten Polyurethandispersion erhältlich ist. Diese Schaumschicht wird auf die zu bedeckende Wunde aufgelegt. Vorteilhafterweise weist dieser Schaum eine mikroporöse, zumindest teilweise offenporige Struktur mit miteinander kommunizierenden Zellen auf.

Die Deckschicht des erfindungsgemäßen Schichtenverbundes umfasst ein thermoplastisches Polymer. Hierunter ist zunächst ein Polymer zu verstehen, welches, wenn es in dem für den Werkstoff für Verarbeitung und Anwendung typischen Temperaturbereich wiederholt erwärmt und abgekühlt werden, thermoplastisch bleibt. Unter thermoplastisch wird die Eigenschaft eines Kunststoffes verstanden, in einem für ihn typischen Temperaturbereich wiederholt in der Wärme zu erweichen und beim Abkühlen zu erhärten und im erweichten Zustand wiederholt durch Fließen zum Beispiel als Formteil, Extrudat oder Umformteil zu Halbzeug oder Gegenständen formbar zu sein. Vorteilhafterweise ist die Deckschicht als semipermeable Membran ausgeführt, also als Membran, welche durch die Schaumschicht hindurch aus der Wunde austretende Flüssigkeit sowie Wasser von Außen zurückhält, aber Wasserdampf passieren lässt.

Dabei hat es sich weiterhin als besonders vorteilhaft erwiesen, wenn bei den Membranen oder Folien ihre Dicken so eingestellt werden, dass diese in einem Bereich von ≥ 5 µm bis ≤ 80 µm, insbesondere von ≥ 5 µm bis ≤ 60 µm und ganz bevorzugt von ≥ 10 µm bis ≤ 30 µm aufweisen und sie eine Reißdehnung von über 450% besitzen.

Im Rahmen der vorliegenden Erfindung sind Deckschicht und Schaumschicht zumindest teilweise direkt miteinander verbunden. Dieses bedeutet, dass es in dem Schichtenverbund Bereiche gibt, in denen die beiden Schichten unmittelbar aufeinander liegen. In diesen Bereichen zeichnet sich der Schichtenverbund gerade dadurch aus, dass kein Klebstoff zwischen der Schaumschicht und der Deckschicht vorhanden ist.

Die Polyurethandispersion (I) umfasst Polyurethane, wobei freie Isocyanatgruppen zumindest teilweise mit anionischen oder potentiell anionischen Hydrophilierungsmitteln umgesetzt wurden. Solche Hydrophilierungsmittel sind Verbindungen, welche gegenüber Isocyanatgruppen reaktive funktionelle Gruppen wie Amino-, Hydroxy- oder Thiolgruppen aufweisen und weiterhin Säureoder Säureaniongruppen wie Carboxylat-, Sulfonat- oder Phosphonatgruppen.

Durch die erfindungsgemäße Auswahl der Schaumschicht und der Deckschicht lassen sich mit einer Schutz-, Deck- und/oder Membranfolie versehene Wundauflagen erhalten, welche durch den Wegfall des Erfordernisses einer Klebschicht in weniger Arbeitsgängen hergestellt werden können. Trotzdem ist die Haftung der Schichten aufeinander ausreichend. Die Schichtenverbunde können weiterhin nach ihrer Herstellung mittels thermischer Umformung weiterverarbeitet werden, so dass eine große Vielfalt an Formen für die Wundauflagen erreichbar ist.

In einer Ausführungsform des erfindungsgemäßen Schichtenverbundes umfasst die Zusammensetzung, aus der der Polyurethanschaum der Schaumschicht erhalten wird, weiterhin Zusatzstoffe, die ausgewählt sind aus der Gruppe umfassend Fettsäureamide, Sulfosuccinamide, Kohlenwasserstoffsulfonate, Kohlenwasserstoffsulfate, Fettsäuresalze, Alkylpolyglycoside und/oder Ethylenoxid/Propylenoxid-Blockcopolymere.

Solche Zusatzstoffe können als Schaumbildner und/oder Schaumstabilisatoren wirken. In den Fettsäureamiden, Sulfosuccinamiden, Kohlenwasserstoffsulfonaten, Kohlenwasserstoffsulfaten oder Fettsäuresalzen enthält der lipophile Rest bevorzugt ≥ 12 bis ≤ 24 Kohlenstoffatome. Geeignete Alkylpolyglycoside sind beispielsweise durch Umsetzung von längerkettigen Monoalkoholen (≥ 4 bis ≤ 22 C-Atome im Alkylrest) mit Mono-, Di- oder Polysacchariden erhältlich. Weiterhin geeignet sind Alkylbenzosulfonate oder Alkylbenzolsulfate mit ≥ 14 bis ≤ 24 Kohlenstoffatomen im Kohlenwasserstoffrest.

Die Fettsäureamide sind vorzugsweise solche auf Basis von Mono- oder Di-(C₂/C₃-alkanol)aminen. Die Fettsäuresalze können beispielsweise Alkalimetallsalze, Aminsalze oder unsubstituierte Ammoniumsalze sein.

Solche Fettsäurederivate basieren typischerweise auf Fettsäuren wie Laurinsäure, Myristinsäure, Palmitinsäure, Ölsäure, Stearinsäure, Ricinolsäure, Behensäure oder Arachidinsäure, Kokosfettsäure, Talgfettsäure, Sojafettsäure und deren Hydrierungsprodukten.

Beispielhaft verwendbare Schaumstabilisatoren sind Mischungen aus Sulfosuccinamiden und Ammoniumstearaten, wobei diese bevorzugt ≥ 20 Gewichts-% bis ≤ 60 Gewichts-%, besonders bevorzugt ≥ 30 Gewichts-% bis ≤ 50 Gewichts-% an Ammoniumstearaten und bevorzugt ≥ 40 Gewichts-%, bis ≤ 80 Gewichts-%, besonders bevorzugt ≥ 50 Gewichts-% bis ≤ 70 Gewichts-% an Sulfosuccinamiden enthalten.

Weitere beispielhaft verwendbare Schaumstabilisatoren sind Mischungen aus Fettalkohol-Polyglykosiden und Ammoniumstearaten, wobei diese bevorzugt ≥ 20 Gewichts-% bis ≤ 60 Gewichts-%, besonders bevorzugt ≥ 30 Gewichts-% bis ≤ 50 Gewichts-% an Ammoniumstearaten und bevorzugt ≥ 40 Gewichts-% bis ≤ 80 Gewichts-%, besonders bevorzugt ≥ 50 Gewichts-% bis ≤ 70 Gewichts-% an Fettalkohol-Polyglykosiden enthalten.

Bei den Ethylenoxid/Propylenoxid-Blockcopolymere handelt es sich um Additionsprodukte von Ethylenoxid und Propylenoxid an OH- oder NH-funktionelle Startermoleküle.

Als Startermoleküle grundsätzlich geeignet sind unter anderem Wasser, Polyethylenglycole, Polypropylenglycole, Glycerin, Trimethylolpropan, Pentaerythrit, Ethylendiamin, Toluylendiamin, Sorbit, Saccharose und Gemische davon.

Bevorzugt werden als Starter di- oder trifunktionelle Verbindungen der vorstehend genannten Art eingesetzt. Besonders bevorzugt sind Polyethylenglycol oder Polypropylenglycol.

Durch die jeweilige Alkylenoxidmenge und die Anzahl von Ethylenoxid (EO)- und Propylenoxid (PO)-Blöcken lassen sich Blockcopolymere unterschiedlicher Art erhalten.

Grundsätzlich ist es auch möglich, dass die an sich streng blockweise aus Ethylenoxid oder Propylenoxid aufgebauten Copolymere auch einzelne Mischblöcke aus EO und PO aufweisen.

Solche Mischblöcke werden erhalten, wenn in die Polyadditionsreaktion Mischungen aus EO und PO eingesetzt werden, so dass bezogen auf diesen Block eine statistische Verteilung von EO und PO in diesem Block resultiert.

Bevorzugt weisen die erfindungsgemäß eingesetzten EO/PO-Blockcopolymere Gehalte an Ethylenoxideinheiten von ≥ 5 Gewichts-%, besonders bevorzugt ≥ 20 Gewichts-% und ganz besonders bevorzugt ≥ 40 Gewichts-% bezogen auf die Summe der im Copolymer vorliegenden Ethylenoxid- und Propylenoxideinheiten auf.

Bevorzugt weisen die erfindungsgemäß eingesetzten EO/PO-Blockcopolymere Gehalte an Ethylenoxideinheiten von ≤ 95 Gewichts-%, besonders bevorzugt ≤ 90 Gewichts-% und ganz besonders bevorzugt ≤ 85 Gewichts-% bezogen auf die Summe der im Copolymer vorliegenden Ethylenoxid-und Propylenoxideinheiten auf.

Bevorzugt weisen die erfindungsgemäß eingesetzten EO/PO-Blockcopolymere zahlenmittlere Molekulargewichte von ≥ 1000 g/mol, besonders bevorzugt ≥ 2000 g/mol, ganz besonders bevorzugt ≥ 5000 g/mol auf.

Bevorzugt weisen die erfindungsgemäß eingesetzten EO/PO-Blockcopolymere zahlenmittlere Molekulargewichte von ≤ 10000 g/mol, besonders bevorzugt ≤ 9500 g/mol, ganz besonders bevorzugt ≤ 9000 g/mol auf.

Vorteilhaft an der Verwendung der EO/PO-Blockcopolymere ist, dass der erhaltene Schaum eine geringere Hydrophobie als bei Verwendung anderer Stabilisatoren aufweist. Hierdurch kann das Aufnahmeverhalten für Flüssigkeiten günstig beeinflusst werden. Zudem werden bei der Verwendung der EO/PO-Blockcopolymere im Gegensatz zu anderen Stabilisatoren nicht-zytotoxische Schäume erhalten.

Es ist bevorzugt, dass die Ethylenoxid/Propylenoxid-Blockcopolymere eine Struktur gemäß der allgemeinen Formel (1) aufweisen: 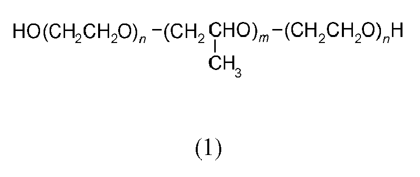 wobei der Wert für n in einem Bereich von ≥ 2 bis ≤ 200 liegt und der Wert für m in einem Bereich von ≥ 10 bis ≤ 60 liegt.

Der Wert für n liegt bevorzugterweise in einem Bereich von ≥ 60 bis ≤ 180, besonders bevorzugt von ≥ 130 bis ≤ 160. Der Wert für m liegt bevorzugterweise in einem Bereich von ≥ 25 bis ≤ 45, besonders bevorzugt von ≥ 25 bis ≤ 35.

Besonders bevorzugt sind EO/PO-Blockcopolymere der vorstehend genannten Art, wobei diese einen hydrophilic-lipophilic-balance (HLB)-Wert von ≥ 4, besonders bevorzugt von ≥ 8 und ganz besonders bevorzugt von ≥ 14 besitzen. Der HLB-Wert errechnet sich nach der Formel HLB = 20 · Mh/M, wobei Mh die zahlenmittlere Molmasse des hydrophilen aus Ethylenoxid gebildeten Anteils des Moleküls und M die zahlenmittlere Molmasse des gesamten Moleküls ist (Griffin, W.C.: Classification of surface active agents by HLB, J. Soc. Cosmet. Chem. 1, 1949). Der HLB-Wert liegt jedoch bei ≤ 19, bevorzugt bei ≤ 18.

In einer Ausführungsform des erfindungsgemäßen Schichtenverbundes ist die wässrige, anionisch hydrophilierte Polyurethandispersion (I) erhältlich, indem
A) isocyanatfunktionelle Prepolymere bereitgestellt werden, welche erhältlich sind aus einer Reaktionsmischung umfassend
   A1) organische Polyisocyanate und
   A2) polymere Polyole mit zahlenmittleren Molekulargewichten von ≥ 400 g/mol bis ≤ 8000 g/mol und OH-Funktionalitäten von ≥ 1,5 bis ≤ 6
   und wobei anschließend
B) die freien NCO-Gruppen der Prepolymere ganz oder teilweise mit
   B1) aminofunktionellen, anionischen oder potentiell anionischen Hydrophilierungsmitteln
   unter Kettenverlängerung umgesetzt werden und die Prepolymere vor, während oder nach Schritt B) in Wasser dispergiert werden, wobei weiterhin in der Reaktionsmischung vorliegende potentiell ionische Gruppen durch teilweise oder vollständige Umsetzung mit einem Neutralisationsmittel in die ionische Form überführt werden.

Bevorzugte wässrige, anionische Polyurethan-Dispersionen (I) haben einen niedrigen Grad an hydrophilen anionischen Gruppen, bevorzugt von ≥ 0,1 bis ≤ 15 Milliequivalenten pro 100 g Festharz.

Um eine gute Sedimentationsstabilität zu erreichen, liegt die zahlenmittlere Teilchengröße der speziellen Polyurethan-Dispersionen bevorzugt bei ≤ 750 nm, besonders bevorzugt bei ≤ 500 nm, bestimmt mittels Laserkorrelations-Spektroskopie.

Das Verhältnis von NCO-Gruppen der Verbindungen aus Komponente A1) zu NCO-reaktiven Gruppen wie Amino-, Hydroxy- oder Thiolgruppen der Verbindungen der Komponenten A2) bis A4) beträgt bei der Herstellung des NCO-funktionellen Prepolymers ≥ 1,05 bis ≤ 3,5, bevorzugt ≥ 1,2 bis ≤ 3,0 besonders bevorzugt ≥ 1,3 bis ≤ 2,5.

Die aminofunktionellen Verbindungen in Stufe B) werden in solch einer Menge eingesetzt, dass das Äquivalentverhältnis von isocyanatreaktiven Aminogruppen dieser Verbindungen zu den freien Isocyanatgruppen des Prepolymers ≥ 40% bis ≤ 150%, bevorzugt zwischen ≥ 50% und ≤ 125%, besonders bevorzugt zwischen ≥ 60% und ≤ 120% beträgt.

Geeignete Polyisocyanate der Komponente A1) sind aromatische, araliphatische, aliphatische oder cycloaliphatische Polyisocyanate mit einer NCO-Funktionalität von ≥ 2.

Beispiele solcher geeigneten Polyisocyanate sind 1,4-Butylendiisocyanat, 1,6-Hexamethylendiisocyanat (HDI), Isophorondiisocyanat (IPDI), 2,2,4- und/oder 2,4,4-Trimethylhexamethylendiisocyanat, die isomeren Bis(4,4'-isocyanatocyclohexyl)methane oder deren Mischungen beliebigen Isomerengehalts, 1,4-Cyclohexylendiisocyanat, 1,4-Phenylendiisocyanat, 2,4- und/oder 2,6-Toluylendiisocyanat (TDI), 1,5-Naphthylendiisocyanat, 2,2'- und/oder 2,4'- und/oder 4,4'-Diphenylmethandiisocyanat (MDI), 1,3- und/oder 1,4-Bis-(2-isocyanato-prop-2-yl)-benzol (TMXDI), 1,3-Bis- (isocyanatomethyl)benzol (XDI), sowie Alkyl-2,6-diisocyanatohexanoate (Lysindiisocyanate) mit C₁- bis C₈-Alkylgruppen.

Neben den vorstehend genannten Polyisocyanaten können anteilig auch modifizierte Diisocyanate mit Uretdion-, Isocyanurat-, Urethan-, Allophanat-, Biuret-, Iminooxadiazindion- und/oder Oxadiazintrionstruktur sowie nicht-modifiziertes Polyisocyanat mit mehr als 2 NCO-Gruppen pro Molekül wie zum Beispiel 4-Isocyanatomethyl-1,8-octandiisocyanat (Nonantriisocyanat) oder Triphenylmethan- 4,4',4"-triisocyanat mit eingesetzt werden.

Bevorzugt handelt es sich um Polyisocyanate oder Polyisocyanatgemische der vorstehend genannten Art mit ausschließlich aliphatisch und/oder cycloaliphatisch gebundenen Isocyanatgruppen und einer mittleren NCO-Funktionalität der Mischung von ≥ 2 bis ≤ 4, bevorzugt ≥ 2 bis ≤ 2,6 und besonders bevorzugt ≥ 2 bis ≤ 2,4.

Besonders bevorzugt werden in A1) 1,6-Hexamethylendiisocyanat, Isophorondiisocyanat, die isomeren Bis(4,4'-isocyanatocyclohexyl)methane sowie deren Mischungen eingesetzt.

In A2) werden polymere Polyole mit einem zahlenmittleren Molekulargewicht Mn von ≥ 400 g/mol bis ≤ 8000 g/mol, bevorzugt von ≥ 400 g/mol bis ≤ 6000 g/mol und besonders bevorzugt von ≥ 600 g/mol bis ≤ 3000 g/mol eingesetzt. Diese weisen bevorzugt eine OH-Funktionalität von ≥ 1,5 bis ≤ 6, besonders bevorzugt von ≥ 1,8 bis ≤ 3, ganz besonders bevorzugt von ≥ 1,9 bis ≤ 2,1 auf.

Solche polymeren Polyole sind beispielsweise Polyesterpolyole, Polyacrylatpolyole, Polyurethanpolyole, Polycarbonatpolyole, Polyetherpolyole, Polyesterpolyacrylatpolyole, Polyurethanpolyacrylatpolyole, Polyurethanpolyesterpolyole, Polyurethanpolyetherpolyole, Polyurethanpolycarbonatpolyole und Polyesterpolycarbonatpolyole. Diese können in A2) einzeln oder in beliebigen Mischungen untereinander eingesetzt werden.

Solche Polyesterpolyole sind Polykondensate aus Di- sowie gegebenenfalls Tri- und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Beispiele für geeignete Diole sind Ethylenglykol, Butylenglykol, Diethylenglykol, Triethylenglykol, Polyalkylenglykole wie Polyethylenglykol, weiterhin 1,2-Propandiol, 1,3-Propandiol, Butandiol(1,3), Butandiol(1,4), Hexandiol(1,6) und Isomere, Neopentylglykol oder Hydroxypivalinsäureneopentylglykolester, wobei Hexandiol(1,6) und Isomere, Neopentylglykol und Hydroxypivalinsäureneopenthylglykolester bevorzugt sind. Daneben können auch Polyole wie Trimethylolpropan, Glycerin, Erythrit, Pentaerythrit, Triemthylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbernsteinsäure, 3,3-Diethylglutarsäure und/oder 2,2-Dimethylbernsteinsäure eingesetzt werden. Als Säurequelle können auch die entsprechenden Anhydride verwendet werden.

Sofern die mittlere Funktionalität des zu veresternden Polyols ≥ 2 ist, können zusätzlich auch Monocarbonsäuren wie Benzoesäure und Hexancarbonsäure mit verwendet werden.

Bevorzugte Säuren sind aliphatische oder aromatische Säuren der vorstehend genannten Art. Besonders bevorzugt sind Adipinsäure, Isophthalsäure und gegebenenfalls Trimellithsäure.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton.

Ebenfalls können in A2) Hydroxylgruppen aufweisende Polycarbonate, bevorzugt Polycarbonatdiole, mit zahlenmittleren Molekulargewichten Mn von ≥ 400 g/mol bis ≤ 8000 g/mol, bevorzugt ≥ 600 g/mol bis ≤ 3000 g/mol eingesetzt werden. Diese sind durch Reaktion von Kohlensäurederivaten wie Diphenylcarbonat, Dimethylcarbonat oder Phosgen mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art.

Bevorzugt enthält das Polycarbonatdiol ≥ 40 Gewichts-% bis ≤ 100 Gewichts-% Hexandiol, bevorzugt 1,6-Hexandiol und/oder Hexandiolderivate. Solche Hexandiolderivate basieren auf Hexandiol und weisen neben endständigen OH-Gruppen auch Ester- oder Ethergruppen auf. Solche Derivate sind durch Reaktion von Hexandiol mit überschüssigem Caprolacton oder durch Veretherung von Hexandiol mit sich selbst zum Di- oder Trihexylenglykol erhältlich.

Statt oder zusätzlich zu reinen Polycarbonatdiolen können auch Polyether-Polycarbonatdiole in A2) eingesetzt werden.

Die Hydroxylgruppen aufweisenden Polycarbonate sind bevorzugt linear gebaut.

Ebenfalls können in A2) Polyetherpolyole eingesetzt werden.

Geeignet sind beispielsweise Polytetramethylenglykolpolyether, wie sie durch Polymerisation von Tetrahydrofuran mittels kationischer Ringöffhung erhältlich sind.

Ebenfalls geeignete Polyetherpolyole sind die Additionsprodukte von Styroloxid, Ethylenoxid, Propylenoxid, Butylenoxid und/oder Epichlorhydrin an di- oder polyfunktionelle Startermoleküle. Polyetherpolyole, basierend auf der zumindest anteiligen Addition von Ethylenoxid an di- oder polyfunktionelle Startermoleküle, können auch als Komponente A4) eingesetzt werden (nichtionische Hydrophilierungsmittel).

Als geeignete Startermoleküle eingesetzt werden können beispielsweise Wasser, Butyldiglykol, Glycerin, Diethylenglykol, Trimethyolpropan, Propylenglykol, Sorbit, Ethylendiamin, Triethanolamin oder 1,4-Butandiol. Bevorzugte Startermoleküle sind Wasser, Ethylenglykol, Propylenglykol, 1,4-Butandiol, Diethylenglykol und Butyldiglykol.

Besonders bevorzugte Ausführungsformen der Polyurethan-Dispersionen (I) enthalten als Komponente A2) eine Mischung aus Polycarbonatpolyolen und Polytetramethylenglykolpolyolen, wobei in dieser Mischung der Anteil an Polycarbonatpolyolen in der Mischung ≥ 20 Gewichts-% bis ≤ 80 Gewichts-% und der Anteil an Polytetramethylenglykolpolyolen ≥ 20 Gewichts-% bis ≤ 80 Gewichts-% beträgt. Bevorzugt ist ein Anteil von ≥ 30 Gewichts-% bis ≤ 75 Gewichts-% an Polytetramethylenglykolpolyolen und ein Anteil von ≥ 25 Gewichts-% bis ≤ 70 Gewichts-% an Polycarbonatpolyolen. Besonders bevorzugt ist ein Anteil von ≥ 35 Gewichts-% bis ≤ 70 Gewichts-% an Polytetramethylenglykolpolyolen und ein Anteil von ≥ 30 Gewichts-% bis ≤ 65 Gewichts-% an Polycarbonatpolyolen, jeweils mit der Maßgabe, dass die Summe der Gewichtsprozente der Polycarbonatpolyole und Polytetramethylenglykolpolyole ≤ 100 Gewichts-% ergibt und der Anteil der Summe der Polycarbonatpolyole und Polytetramethylenglykolpolyetherpolyole an der Komponente A2) ≥ 50 Gewichts-%, bevorzugt ≥ 60 Gewichts-% und besonders bevorzugt ≥ 70 Gewichts-% beträgt.

Unter isocyanatreaktiven anionischen oder potentiell anionischen Hydrophilierungsmitteln der Komponente B1) werden sämtliche Verbindungen verstanden, die mindestens eine isocyanatreaktive Gruppe wie eine Amino-, Hydroxy- oder Thiolgruppe aufweisen, sowie mindestens eine Funktionalität wie zum Beispiel -COO⁻M⁺, -SO₃⁻M⁺, -PO(O⁻M⁺)₂ mit M⁺ beispielsweise gleich Metallkation, H⁺, NH₄⁺, NHR₃⁺, wobei R jeweils ein C₁-C₁₂-Alkylrest, C₅-C₆-Cycloalkylrest und/oder ein C₂-C₄-Hydroxyalkylrest sein kann, die bei Wechselwirkung mit wässrigen Medien ein pH-Wert-abhängiges Dissoziationsgleichgewicht eingeht und auf diese Weise negativ oder neutral geladen sein kann.

Vorzugsweise sind die isocyanatreaktiven anionischen oder potentiell anionischen Hydrophilierungsmittel isocyanatreaktive aminofunktionelle anionische oder potentiell anionische Hydrophilierungsmittel.

Geeignete anionisch oder potentiell anionisch hydrophilierende Verbindungen sind Mono- und Diaminocarbonsäuren, Mono- und Diaminosulfonsäuren sowie Mono- und Diaminophosphonsäuren und ihre Salze. Beispiele solcher anionischen oder potentiell anionischen Hydrophilierungsmittel sind N-(2-Aminoethyl)-β-alanin, 2-(2-Amino-ethylamino)-ethansulfonsäure, Ethylendiaminpropyl- oder -butylsulfonsäure, 1,2- oder 1,3-Propylendiamin-β-ethylsulfonsäure, Glycin, Alanin, Taurin, Lysin, 3,5-Diaminobenzoesäure und das Additionsprodukt von IPDA und Acrylsäure (EP-A 0 916 647, Beispiel 1). Weiterhin kann Cyclohexylaminopropansulfonsäure (CAPS) aus WO-A 0/88006 als anionisches oder potentiell anionisches Hydrophilierungsmittel verwendet werden.

Bevorzugte anionische oder potentiell anionische Hydrophilierungsmittel der Komponente B1) sind solche der vorstehend genannten Art, die über Carboxylat- beziehungsweise Carobonsäuregruppen und/oder Sulfonatgruppen verfügen, wie die Salze von N-(2-Aminoethyl)-β-alanin, der 2-(2-Aminoethylamino)ethansulfonsäure oder des Additionsproduktes von IPDA und Acrylsäure (EP-A 0 916 647, Beispiel 1).

Zur Hydrophilierung können auch Mischungen aus anionischen beziehungsweise potentiell anionischen Hydrophilierungsmitteln und nichtionischen Hydrophilierungsmitteln verwendet werden.

In einer weiteren Ausführungsform des erfindungsgemäßen Schichtenverbundes umfasst die Reaktionsmischung in Schritt A) weiterhin:
A3) hydroxyfunktionelle Verbindungen mit Molekulargewichten von ≥ 62 g/mol bis ≤ 399 g/mol

Die Verbindungen der Komponente A3) besitzen Molekulargewichte von ≥ 62 g/mol bis ≤ 399 g/mol.

In A3) können Polyole des genannten Molekulargewichtsbereichs mit bis zu 20 Kohlenstoffatomen, wie Ethylenglykol, Diethylenglykol, Triethylenglykol, 1,2-Propandiol, 1,3-Propandiol, 1,4- Butandiol, 1,3-Butylenglykol, Cyclohexandiol, 1 ,4-Cyclohexandimethanol, 1,6-Hexandiol, Neopentylglykol, Hydrochinondihydroxyethylether, Bisphenol A (2,2-Bis(4-hydroxyphenyl)-propan), hydriertes Bisphenol A, (2,2-Bis(4-hydroxycyclohexyl)propan), Trimethylolpropan, Glycerin, Pentaerythrit sowie deren beliebige Mischungen untereinander eingesetzt werden.

Geeignet sind auch Esterdiole des genannten Molekulargewichtsbereichs wie αHydroxybutyl-8-hydroxycapronsäureester, (ω-Hydroxyhexyl-γ-hydroxybuttersäureester, Adipinsäure-(β-hydroxyethyl)ester oder Terephthalsäurebis(β-hydroxyethyl)-ester.

Ferner können in A3) auch monofunktionelle, isocyanatreaktive, Hydroxylgruppenhaltige Verbindungen eingesetzt werden. Beispiele solcher monofunktionellen Verbindungen sind Ethanol, n-Butanol, Ethylenglykolmonobutylether, Diethylenglykolmonomethylether, Ethylenglykolmonobutylether, Diethylenglykolmonobutylether, Propylenglykolmonomethylether, Dipropylenglykolmonomethylether, Tripropylenglykolmonomethylether, Dipropylenglykolmonopropylether, Propylenglykolmonobutylether, Dipropylenglykolmonobutylether, Tripropylenglykolmonobutylether, 2- Ethylhexanol, 1-Octanol, 1-Dodecanol, 1-Hexadecanol.

Bevorzugte Verbindungen der Komponente A3) sind 1,6-Hexandiol, 1,4-Butandiol, Neopentylglykol und Trimethylolpropan.

In einer weiteren Ausführungsform des erfindungsgemäßen Schichtenverbundes umfasst die Reaktionsmischung in Schritt A) weiterhin:
A4) isocyanatreaktive, anionische oder potentiell anionische und gegebenenfalls nichtionische Hydrophilierungsmittel

Unter anionisch beziehungsweise potentiell anionisch hydrophilierenden Verbindungen der Komponente A4) werden sämtliche Verbindungen verstanden, die mindestens eine isocyanatreaktive Gruppe wie eine Amino-, Hydroxyl- oder Thiolgruppe aufweisen sowie mindestens eine Funktionalität wie zum Beispiel -COO⁻M⁺, -SO₃⁻M⁺, -PO(O⁻M⁺)₂ mit M⁺ beispielsweise gleich Metallkation, H⁺, NH₄⁺, NHR₃⁺, wobei R jeweils ein C₁-C₁₂-Alkylrest, C₅-C₆-Cycloalkylrest und/oder ein C₂-C₄-Hydroxyalkylrest sein kann, die bei Wechselwirkung mit wässrigen Medien ein pH-Wert-abhängiges Dissoziationsgleichgewicht eingeht und auf diese Weise negativ oder neutral geladen sein kann. Geeignete anionisch oder potentiell anionisch hydrophilierende Verbindungen sind zum Beispiel Mono- und Dihydroxycarbonsäuren, Mono- und Dihydroxysulfonsäuren sowie Mono- und Dihydroxyphosphonsäuren und ihre Salze. Beispiele solcher anionischen beziehungsweise potentiell anionischen Hydrophilierungsmittel sind Dimethylolpropionsäure, Dimethylolbuttersäure, Hydroxypivalinsäure, Äpfelsäure, Zitronensäure, Glykolsäure, Milchsäure und das propoxylierte Addukt aus 2-Butendiol und NaHSO₃, wie es in DE-A 2 446 440, Seite 5 - 9, Formel I-III beschrieben ist. Bevorzugte anionische oder potentiell anionische Hydrophilierungsmittel der Komponente A4) sind solche der vorstehend genannten Art, die über Carboxylat- beziehungsweise Carbonsäuregruppen und/oder Sulfonatgruppen verfügen.

Besonders bevorzugte anionische oder potentiell anionische Hydrophilierungsmittel sind solche, die Carboxylat- beziehungsweise Carbonsäuregruppen als ionische oder potentiell ionische Gruppen enthalten wie Dimethylolpropionsäure, Dimethylolbuttersäue und Hydroxypivalinsäure und/oder deren Salze.

Geeignete nichtionisch hydrophilierende Verbindungen der Komponente A4) sind zum Beispiel Polyoxyalkylenether, die mindestens eine Hydroxy- oder Aminogruppe, bevorzugt mindestens eine Hydroxygruppe enthalten. Beispiele hierfür sind die monohydroxyfunktionellen, im statistischen Mittel ≥ 5 bis ≤ 70, bevorzugt ≥ 7 bis ≤ 55 Ethylenoxideinheiten pro Molekül aufweisenden Polyalkylenoxidpolyetheralkohole, wie durch Alkoxylierung geeigneter Startermoleküle zugänglich sind. Diese sind entweder reine Polyethylenoxidether oder gemischte Polyalkylenoxidether, wobei sie ≥ 30 mol-%, bevorzugt ≥ 40 mol-% bezogen auf alle enthaltenen Alkylenoxideinheiten an Ethylenoxideinheiten enthalten.

Bevorzugte Polyethylenoxidether der vorstehend genannten Art sind monofunktionelle gemischte Polyalkylenoxidpolyether, die ≥ 40 mol-% bis ≤ 100 mol-% Ethylenoxid- und ≥ 0 mol-% bis ≤ 60 mol-% Propylenoxideinheiten aufweisen.

Bevorzugte nichtionisch hydrophilierende Verbindungen der Komponente A4) sind solche der vorstehend genannten Art, wobei es sich um Block(co)polymere handelt, die durch blockweise Addition von Alkylenoxiden an geeignete Starter hergestellt werden.

Geeignete Startermoleküle für solche nichtionischen Hydrophilierungsmittel sind gesättigte Monoalkohole wie Methanol, Ethanol, n-Propanol, Isopropanol, n-Butanol, Isobutanol, sec-Butanol, die isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykolmonoalkylether, wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole der vorstehend genannten Art. Besonders bevorzugt werden Diethylenglykolmonobutylether oder n-Butanol als Startermoleküle verwendet.

Für die Alkoxylierungsreaktion geeignete Alkylenoxide sind insbesondere Ethylenoxid und Propylenoxid, die in beliebiger Reihenfolge oder auch im Gemisch bei der Alkoxylierungsreaktion eingesetzt werden können.

In einer weiteren Ausführungsform des erfindungsgemäßen Schichtenverbundes werden in Schritt B) die freien NCO-Gruppen der Prepolymere weiterhin ganz oder teilweise umgesetzt mit
B2) aminofunktionelle Verbindungen mit Molekulargewichten von ≥ 32 g/mol bis ≤ 400 g/mol

Als Komponente B2) können Di- oder Polyamine wie 1,2-Ethylendiamin, 1,2- und 1,3-Diaminopropan, 1,4-Diaminobutan, 1,6-Diaminohexan, Isophorondiamin, Isomerengemische von 2,2,4-und 2,4,4-Trimethylhexamethylendiamin, 2-Methylpentamethylendiamin, Diethylentriamin, Triaminononan, 1,3- und 1,4-Xylylendiamin, α,α,α',α'-Tetramethyl-1,3- und -1,4-xylylendiamin und 4,4-Diaminodicyclohexylmethan und/oder Dimethylethylendiamin eingesetzt werden. Ebenfalls möglich, aber weniger bevorzugt, ist die Verwendung von Hydrazin sowie Hydraziden wie Adipinsäuredihydrazid.

Darüber hinaus können als Komponente B2) auch Verbindungen, die neben einer primären Aminogruppe auch sekundäre Aminogruppen oder neben einer Aminogruppe (primär oder sekundär) auch OH-Gruppen aufweisen, eingesetzt werden. Beispiele hierfür sind primäre/sekundäre Amine, wie Diethanolamin, 3-Amino-1-methylaminopropan, 3-Amino-1-ethylaminopropan, 3-Amino-1-cyclohexylaminopropan, 3-Amino-1-Methylaminobutan, Alkanolamine wie N-Aminoethylethanolamin, Ethanolamin, 3-Aminopropanol, Neopentanolamin.

Ferner können als Komponente B2) auch monofunktionelle isocyanatreaktive Aminverbindungen eingesetzt werden, wie beispielsweise Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin oder geeignete substituierte Derivate davon, Amidamine aus diprimären Aminen und Monocarbonsäuren, Monoketime von diprimären Aminen, primär/tertiäre Amine, wie N,N-Dimethylaminopropylamin. Bevorzugte Verbindungen der Komponente B2) sind 1,2-Ethylendiamin, 1,4-Diaminobutan und Isophorondiamin.

In einer weiteren Ausführungsform des erfindungsgemäßen Schichtenverbundes ist bei der Herstellung der wässrigen, anionisch hydrophilierten Polyurethan-Dispersionen (I) die Komponente A1) ausgewählt aus der Gruppe umfassend 1,6-Hexamethylendiisocyanat, Isophorondiisocyanat und/oder die isomeren Bis-(4,4'-isocyanatocyclohexyl)methane. Weiterhin umfasst die Komponente A2) eine Mischung aus Polycarbonatpolyolen und Polytetramethylenglykolpolyolen, wobei der Anteil der Summe der Polycarbonatpolyole und der Polytetramethylenglykolpolyetherpolyole an der Komponente A2) ≥ 70 Gewichts-% bis ≤ 100 Gewichts-% beträgt.

Neben den Polyurethan-Dispersionen (I) und den Zusatzstoffen können auch weitere Hilfsstoffe verwendet werden.

Beispiele für solche Hilfsstoffe sind Verdicker beziehungsweise Thixotropiermittel, Antioxidantien, Lichtschutzmittel, Emulgatoren, Weichmacher, Pigmente, Füllstoffe und/oder Verlaufshilfsmittel.

Als Verdicker können handelsübliche Verdicker eingesetzt werden, wie Dextrin-, Stärke- oder Cellulosederivate wie Celluloseether oder Hydroxyethylcellulose, Polysaccharidderivate wie Gummi arabicum oder Guar, organische vollsynthetische Verdicker auf Basis von Polyacrylsäuren, Polyvinylpyrrolidonen, Poly(meth)acrylverbindungen oder Polyurethanen (assoziative Verdicker) sowie anorganische Verdicker wie Bentonite oder Kieselsäuren.

Grundsätzlich können die erfindungsgemäßen Zusammensetzungen auch Vernetzer wie unblockierte Polyisocyanate, Amid- und Amin-Formaldehydharze, Phenolharze, Aldehyd- und Ketonharze, wie zum Beispiel Phenol-Formaldehydharze, Resole, Furanharze, Harnstoffharze, Carbamidsäureesterharze, Triazinharze, Melaminharze, Benzoguanaminharze, Cyanamidharze oder Anilinharze enthalten.

In einer weiteren Ausführungsform des erfindungsgemäßen Schichtenverbundes umfasst das thermoplastische Polymer der Deckschicht Materialien, die ausgewählt sind aus der Gruppe umfassend Polyurethan, Polyethylen, Polypropylen, Polyvinylchlorid, Polystyrol, Polyether, Polyester, Polyamid, Polycarbonat, Polyether-Polyamid-Copolymeren, Polyacrylat, Polymethacrylat und/oder Polymaleat. Vorzugsweise handelt es sich hierbei um thermoplastisches Polyurethan (TPU). Vorteilhafterweise sind diese Materialien elastomer. Günstig sind hierbei auch Folien aus solchen Materialien, die eine Dicke von ≥ 5 µm bis ≤ 80 µm, insbesondere von ≥ 5 µm bis ≤ 60 µm und ganz bevorzugt von ≥ 10 µm bis ≤ 30 µm aufweisen.

In einer weiteren Ausführungsform des erfindungsgemäßen Schichtenverbundes umfasst das thermoplastische Polymer der Deckschicht Polyurethane, die ausgewählt sind aus der Gruppe umfassend aliphatische Polyesterpolyurethane, aromatische Polyesterpolyurethane, aliphatische Polyetherpolyurethane und/oder aromatische Polyetherpolyurethane. Somit lassen sich atmungsaktive elastische Membranfolien erhalten. Die so erhältlichen Deckschichen zeichnen sich aus durch hohe Flexibilität/Elastizität über einen breiten Temperaturbereich, gute Wind- und Wasserdichtigkeit bei gleichzeitig hoher Wasserdampfdurchlässigkeit, Geräuscharmut, gute textile Haptik, Wasch- und Reinigungsbeständigkeit, sehr gute chemische und mechanische Beständigkeit und Weichmacherfreiheit.

In einer weiteren Ausführungsform des erfindungsgemäßen Schichtenverbundes weist die direkte Verbindung zwischen der Schaumschicht und der Deckschicht eine Schälfestigkeit von ≥ 0,01 N/mm bis ≤ 0,50 N/mm auf. Die Schälfestigkeit kann auch in einem Bereich von ≥ 0,03 N/mm bis ≤ 0,30 N/mm oder von ≥ 0,05 N/mm bis ≤ 0,20 N/mm liegen. Man kann die Schälfestigkeit ermitteln, indem an einer Zwick Universalprüfmaschine 360° Peel-Prüfungen mit einer Traversengeschwindigkeit von 100 mm/min durchgeführt werden.

In einer weiteren Ausführungsform des erfindungsgemäßen Schichtenverbundes beträgt die Durchlässigkeit des Schichtenverbundes gegenüber Wasserdampf ≥ 1000 g / 24 h × m² bis ≤ 4000 g / 24 h × m². Diese Durchlässigkeit gegenüber Wasserdampf kann auch in einem Bereich von ≥ 1500 g / 24 h × m² bis ≤ 3000 g / 24 h × m² oder von ≥ 1800 g / 24 h × m² bis ≤ 2500 g / 24 h × m² liegen.

Es ist möglich, dass in dem erfindungsgemäßen Schichtenverbundes die Deckschicht eine Dichtigkeit gegenüber Wasser, ausgedrückt als auf der Schicht lastende Wassersäule, von ≥ 2000 mm aufweist. Dieser Wert kann auch in einem Bereich von ≥ 4000 mm oder von ≥ 6000 mm liegen.

Weiterhin kann die Deckschicht eine Durchlässigkeit gegenüber Wasserdampf von ≥ 1000 g / 24 h × m² bis ≤ 8000 g / 24 h × m² aufweisen. Diese Durchlässigkeit gegenüber Wasserdampf kann auch in einem Bereich von ≥ 2000 g / 24 h × m² bis ≤ 6000 g / 24 h × m² oder von ≥ 3000 g / 24 h × m² bis ≤ 5000 g / 24 h × m² liegen.

In einer beispielhaften Rezeptur zur Herstellung der Polyurethan-Dispersionen werden die Komponenten A1) bis A4) und B1) bis B2) in den folgenden Mengen eingesetzt, wobei sich die Einzelmengen stets zu ≤ 100 Gewichts-% addieren:
≥ 5 Gewichts-% bis ≤ 40 Gewichts-% der Komponente A1);
≥ 55 Gewichts-% bis ≤ 90 Gewichts-% der Komponente A2);
≥ 0,5 Gewichts-% bis ≤ 20 Gewichts-% Summe der Komponenten A3) und B2);
≥ 0,1 Gewichts-% bis ≤ 25 Gewichts-% Summe der Komponenten A4) und B1), wobei bezogen auf die Gesamtmengen der Komponenten A1) bis A4) und B1) bis B2) ≥ 0,1 Gewichts-% bis ≤ 5 Gewichts-% an anionischen oder potentiell anionischen Hydrophilierungsmitteln aus A4) und/oder B1) verwendet werden.

In einer weiteren beispielhaften Rezeptur zur Herstellung der Polyurethan-Dispersionen werden die Komponenten A1) bis A4) und B1) bis B2) in den folgenden Mengen eingesetzt, wobei sich die Einzelmengen stets zu ≤ 100 Gewichts-% aufaddieren:
≥ 5 Gewichts-% bis ≤ 35 Gewichts-% der Komponente A1);
≥ 60 Gewichts-% bis ≤ 90 Gewichts-% der Komponente A2);
≥ 0,5 Gewichts-% bis ≤ 15 Gewichts-% Summe der Komponenten A3) und B2);
≥ 0,1 Gewichts-% bis ≤ 15 Gewichts-% Summe der Komponenten A4) und B1), wobei bezogen auf die Gesamtmengen der Komponenten A1) bis A4) und B1) bis B2) ≥ 0,2 Gewichts-% bis ≤ 4 Gewichts-% an anionischen bzw. potentiell anionischen Hydrophilierungsmitteln aus A4) und/oder B1) verwendet werden.

In einer ganz besonders bevorzugten Rezeptur zur Herstellung der Polyurethan-Dispersionen werden die Komponenten A1) bis A4) und B1) bis B2) in den folgenden Mengen eingesetzt, wobei sich die Einzelmengen stets zu ≤ 100 Gewichts-% aufaddieren:
≥ 10 Gewichts-% bis ≤ 30 Gewichts-% der Komponente A1);
≥ 65 Gewichts-% bis ≤ 85 Gewichts-% der Komponente A2);
≥ 0,5 Gewichts-% bis ≤ 14 Gewichts-% Summe der Komponenten A3) und B2);
≥ 0,1 Gewichts-% bis ≤ 13,5 Gewichts-% Summe der Komponenten A4) und B1), wobei bezogen auf die Gesamtmengen der Komponenten A1) bis A4) und B1) bis B2) ≥ 0,5 Gewichts-% bis ≤ 3,0 Gewichts-% an anionischen oder potentiell anionischen Hydrophilierungsmitteln aus A4) und/oder B1) verwendet werden.

Die Herstellung der anionisch hydrophilierten Polyurethan-Dispersionen (I) kann in einer oder mehreren Stufe/-n in homogener oder bei mehrstufiger Umsetzung, teilweise in disperser Phase durchgeführt werden. Nach vollständig oder teilweise durchgeführter Polyaddition aus A1) bis A4) erfolgt ein Dispergier-, Emulgier- oder Lösungsschritt. Im Anschluss erfolgt gegebenenfalls eine weitere Polyaddition oder Modifikation in disperser Phase.

Dabei können Verfahren wie beispielsweise Prepolymer- Mischverfahren, Acetonverfahren oder Schmelzdispergierverfahren verwendet werden. Bevorzugt wird das Aceton-Verfahren verwendet.

Für die Herstellung nach dem Aceton-Verfahren werden üblicherweise die Bestandteile A2) bis A4) und die Polyisocyanatkomponente A1) zur Herstellung eines isocyanatfunktionellen Polyurethan-Prepolymers ganz oder teilweise vorgelegt und gegebenenfalls mit einem mit Wasser mischbaren aber gegenüber Isocyanatgruppen inerten Lösungsmittel verdünnt und auf Temperaturen im Bereich von ≥ 50 °C bis ≤ 120 °C aufgeheizt. Zur Beschleunigung der Isocyanatadditionsreaktion können die in der Polyurethan-Chemie bekannten Katalysatoren eingesetzt werden.

Geeignete Lösungsmittel sind die üblichen aliphatischen, ketofunktionellen Lösemittel wie Aceton oder 2-Butanon, die nicht nur zu Beginn der Herstellung, sondern gegebenenfalls in Teilen auch später zugegeben werden können. Bevorzugt sind Aceton und 2-Butanon.

Andere Lösemittel wie Xylol, Toluol, Cyclohexan, Butylacetat, Methoxypropylacetat, N-Methylpyrrolidon, N-Ethylpyrrolidon, Lösemittel mit Ether- oder Estereinheiten können zusätzlich eingesetzt und ganz oder teilweise abdestilliert werden oder vollständig im Falle von N-Methylpyrrolidon, N-Ethylpyrrolidon in der Dispersion verbleiben. Bevorzugt werden aber außer den üblichen aliphatischen, ketofunktionellen Lösemitteln keine anderen Lösungsmittel verwendet.

Anschließend werden die gegebenenfalls zu Beginn der Reaktion noch nicht zugegebenen Bestandteile von A1) bis A4) zudosiert.

Bei der Herstellung des Polyurethan-Prepolymeren aus A1) bis A4) beträgt das Stoffmengenverhältnis von Isocyanatgruppen zu mit isocyanatreaktiven Gruppen beispielsweise ≥ 1,05 bis ≤ 3,5, bevorzugt ≥ 1,2 bis ≤ 3,0 und besonders bevorzugt ≥ 1,3 bis ≤ 2,5.

Die Umsetzung der Komponenten A1) bis A4) zum Prepolymer erfolgt teilweise oder vollständig, bevorzugt aber vollständig. Es werden so Polyurethan-Prepolymere, die freie Isocyanatgruppen enthalten, in Substanz oder in Lösung erhalten.

Im Neutralisationsschritt zur teilweisen oder vollständigen Überführung potentiell anionischer Gruppen in anionische Gruppen werden Basen wie tertiäre Amine, zum Beispiel Trialkylamine mit ≥ 1 bis ≤ 12, bevorzugt ≥ 1 bis ≤ 6 C-Atomen, besonders bevorzugt ≥ 2 bis ≤ 3 C-Atomen in jedem Alkylrest oder Alkalimetallbasen wie die entsprechenden Hydroxide eingesetzt.

Beispiele hierfür sind Trimethylamin, Triethylamin, Methyldiethylamin, Tripropylamin, N-Methylmorpholin, Methyldiisopropylamin, Ethyldiisopropylamin und Diisopropylethylamin. Die Alkylreste können beispielsweise auch Hydroxylgruppen tragen, wie bei den Dialkylmonoalkanol-, Alkyldialkanol- und Trialkanolaminen. Als Neutralisationsmittel sind gegebenenfalls auch anorganische Basen, wie wässrige Ammoniaklösung oder Natrium- bzw. Kaliumhydroxid einsetzbar.

Bevorzugt sind Ammoniak, Triethylamin, Triethanolamin, Dimethylethanolamin oder Diisopropylethylamin sowie Natriumhydroxid und Kaliumhydroxid, besonders bevorzugt sind Natriumhydroxid und Kaliumhydroxid.

Die Stoffmenge der Basen beträgt zwischen ≥ 50 mol-% und ≤ 125 mol%, bevorzugt zwischen ≥ 70 mol-% und ≤ 100 mol% der Stoffmenge der zu neutralisierenden Säuregruppen. Die Neutralisation kann auch gleichzeitig mit der Dispergierung erfolgen, indem das Dispergierwasser bereits das Neutralisationsmittel enthält.

Im Anschluss wird in einem weiteren Verfahrensschritt, falls noch nicht oder nur teilweise geschehen das erhaltene Prepolymer mit Hilfe von aliphatischen Ketonen wie Aceton oder 2-Butanon gelöst.

Bei der Kettenverlängerung in Stufe B) werden NH₂- und/oder NH-funktionelle Komponenten mit den noch verbliebenen Isocyanatgruppen des Prepolymers teilweise oder vollständig umgesetzt. Bevorzugt wird die Kettenverlängerung vor der Dispergierung in Wasser durchgeführt.

Zur Kettenterminierung werden üblicherweise Amine B2) mit einer gegenüber Isocyanaten reaktiven Gruppe wie Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketime von diprimären Aminen, primär/tertiäre Amine, wie N,N-Dimethylaminopropylamin verwendet.

Werden zur teilweisen oder vollständigen Kettenverlängerung anionische oder potentiell anionische Hydrophilierungsmittel entsprechend der Definition B1) mit NH₂- oder NH-Gruppen eingesetzt, erfolgt die Kettenverlängerung der Prepolymere bevorzugt vor der Dispergierung.

Die aminischen Komponenten B1) und B2) können gegebenenfalls in wasser- oder lösemittelverdünnter Form im erfindungsgemäßen Verfahren einzeln oder in Mischungen eingesetzt werden, wobei grundsätzlich jede Reihenfolge der Zugabe möglich ist.

Wenn Wasser oder organische Lösemittel als Verdünnungsmittel mit verwendet werden, so beträgt der Verdünnungsmittelgehalt in der in B) eingesetzten Komponente zur Kettenverlängerung bevorzugt ≥ 70 Gewichts-% bis ≤ 95 Gewichts-%.

Die Dispergierung erfolgt bevorzugt im Anschluss an die Kettenverlängerung. Dazu wird das gelöste und kettenverlängerte Polyurethanpolymer gegebenenfalls unter starker Scherung, wie zum Beispiel starkem Rühren, entweder in das Dispergierwasser eingetragen oder es wird umgekehrt das Dispergierwasser zu den kettenverlängerte Polyurethanpolymerlösungen gerührt. Bevorzugt wird das Wasser in das gelöste kettenverlängerte Polyurethanpolymer gegeben.

Das in den Dispersionen nach dem Dispergierschritt noch enthaltene Lösemittel wird üblicherweise anschließend destillativ entfernt. Eine Entfernung bereits während der Dispergierung ist ebenfalls möglich.

Der Restgehalt an organischen Lösemitteln in den Polyurethan-Dispersionen (I) beträgt typischerweise ≤ 1,0 Gew.-%, bevorzugt ≤ 0,5 Gew.-%, bezogen auf die gesamte Dispersion.

Der pH-Wert der erfindungsgemäßen Polyurethan-Dispersionen (I) beträgt typischerweise ≤ 9,0, bevorzugt ≤ 8,5, besonders bevorzugt weniger als ≤ 8,0 und liegt ganz besonders bevorzugt bei ≥ 6,0 bis ≤ 7,5.

Der Feststoffgehalt der Polyurethan-Dispersionen (I) beträgt bevorzugt ≥ 40 Gewichts-% bis ≤ 70 Gewichts-%, besonders bevorzugt ≥ 50 Gewichts-% bis ≤ 65 Gewichts-%, ganz besonders bevorzugt ≥ 55 Gewichts-% bis ≤ 65 Gewichts-% und insbesondere ≥ 60 Gewichts-% bis ≤ 65 Gewichts-%.

Beispiele für erfindungsgemäße Zusammensetzungen werden nachfolgend aufgeführt, wobei die Summe der Angaben in Gewichts-% einen Wert von ≤ 100 Gewichts-% einnimmt. Diese Zusammensetzungen umfassen, bezogen auf die Trockensubstanz, typischerweise ≥ 80 Gewichtsteile bis ≤ 99,5 Gewichtsteile der Dispersion (I), ≥ 0 Gewichtsteile bis ≤ 10 Gewichtsteile Schaumhilfsmittel, ≥ 0 Gewichtsteile bis ≤ 10 Gewichtsteile Vernetzer und ≥ 0 Gewichtsteile bis ≤ 10 Gewichtsteile Verdicker.

Bevorzugt umfassen diese erfindungsgemäßen Zusammensetzungen, bezogen auf die Trockensubstanz, ≥ 85 Gewichtsteile bis ≤ 97 Gewichtsteile der Dispersion (I), ≥ 0,5 Gewichtsteile bis ≤ 7 Gewichtsteile Schaumhilfsmittel, ≥ 0 Gewichtsteile bis ≤ 5 Gewichtsteile Vernetzer und ≥ 0 Gewichtsteile bis ≤ 5 Gewichtsteile Verdicker.

Besonders bevorzugt umfassen diese erfindungsgemäßen Zusammensetzungen, bezogen auf die Trockensubstanz, ≥ 89 Gewichtsteile bis ≤ 97 Gewichtsteile der Dispersion (I), ≥ 0,5 Gewichtsteile bis ≤ 6 Gewichtsteile Schaumhilfsmittel, ≥ 0 Gewichtsteile bis ≤ 4 Gewichtsteile Vernetzer und ≥ 0 Gewichtsteile bis ≤ 4 Gewichtsteile Verdicker.

Beispiele für erfindungsgemäße Zusammensetzungen, welche Ethylenoxid/Propylenoxid-Blockcopolymere als Schaumstabilisatoren umfassen, werden nachfolgend aufgeführt. Diese Zusammensetzungen umfasse, bezogen auf Trockensubstanz, ≥ 80 Gewichtsteile bis ≤ 99,9 Gewichtsteile der Dispersion (I) und ≥ 0,1 Gewichtsteile bis ≤ 20 Gewichtsteile der Ethylenoxid/Propylenoxid-Blockcopolymere. Bevorzugt umfassen die Zusammensetzungen, bezogen auf Trockensubstanz, ≥ 85 Gewichtsteile bis ≤ 99,5 Gewichtsteile der Dispersion (I) und 0,5 bis 15 Gewichtsteile der Ethylenoxid/Propylenoxid-Blockcopolymere. Besonders bevorzugt sind hierbei ≥ 90 Gewichtsteile bis ≤ 99 Gewichtsteile der Dispersion (I) und ≥ 1 Gewichtsteile bis ≤ 10 Gewichtsteile der Ethylenoxid/Propylenoxid-Blockcopolymere und ganz besonders bevorzugt ≥ 94 Gewichtsteile bis ≤ 99 Gewichtsteile der Dispersion (I) und ≥ 1 bis ≤ 6 Gewichtsteile der Ethylenoxid/Propylenoxid-Blockcopolymere.

Im Sinne der vorliegenden Erfindung bedeutet die Angabe "Gewichtsteile" einen relativen Anteil, jedoch nicht im Sinne der Angabe von Gewichts-%. Folglich kann die zahlenmäßige Summe der Gewichtsanteile auch Werte über 100 annehmen.

Neben den genannten Komponenten können in den erfindungsgemäßen Zusammensetzungen auch weitere wässrige Bindemittel eingesetzt werden. Solche wässrigen Bindemittel können zum Beispiel aus Polyester-, Polyacrylat-, Polyepoxid- oder anderen Polyurethanpolymeren aufgebaut sein. Auch die Kombination mit strahlenhärtbaren Bindemitteln, wie sie beispielsweise in der EP-A-0 753 531 beschrieben sind, ist möglich. Ferner können auch andere anionische oder nichtionische Dispersionen, wie Polyvinylacetat-, Polyethylen-, Polystyrol-, Polybutadien-, Polyvinylchlorid-, Polyacrylat- und Copolymerisat-Dispersionen eingesetzt werden.

Das Aufschäumen im erfindungsgemäßen Verfahren geschieht durch mechanisches Rühren der Zusammensetzung bei hohen Drehzahlen, durch Schütteln oder durch Entspannung eines Treibgases.

Das mechanische Aufschäumen kann mit beliebigen mechanischen Rühr-, Misch- und Dispergiertechniken erfolgen. In der Regel wird hierbei Luft eingetragen, aber auch Stickstoff und andere Gase können hierfür benutzt werden.

Gegenstand der Erfindung ist weiterhin ein Verfahren zur Herstellung eines Schichtenverbundes gemäß der vorliegenden Erfindung, umfassend die Schritte:
- Bereitstellen einer Schaumschicht, welche einen Polyurethanschaum umfasst, der erhalten wird, indem eine Zusammensetzung umfassend eine wässrige, anionisch hydrophilierte Polyurethandispersion (I) aufgeschäumt wird;
- Aufbringen einer Deckschicht auf die Schaumschicht.

Der erhaltene Schaum kann beim Aufschäumen oder unmittelbar danach auf ein Substrat aufgetragen oder in eine Form gegeben und getrocknet werden. Als Substrate geeignet sind insbesondere Papiere oder Folien, die ein einfaches Ablösen der Wundauflage vor ihrem Einsatz zur Abdeckung einer verletzten Stelle ermöglichen.

Der Auftrag kann beispielsweise durch Gießen oder Rakeln erfolgen, aber auch andere an sich bekannte Techniken sind möglich. Ein mehrschichtiger Auftrag mit zwischengeschalteten Trocknungsschritten ist grundsätzlich auch möglich. Hieran kann sich dann das Aufbringen der Deckschicht anschließen, gefolgt von einer Trocknung des Schichtenverbundes.

Es ist alternativ möglich, dass die Schaumschicht direkt auf die Deckschicht gerakelt wird und der noch feuchte Schichtenverbund getrocknet wird.

Weiterhin ist es alternativ möglich, dass auf die zuvor getrocknete Schaumschicht die Deckschicht oder auf Deckschicht die zuvor getrocknete Schaumschicht aufgelegt wird und der erhaltene Schichtenverbund noch getempert wird.

Vorteilhafterweise aber wird das Aufbringen der Deckschicht auf die Schaumschicht derart ausgeführt wird, dass die Deckschicht auf die zuvor getrocknete Schaumschicht auflaminiert wird. Zum Auflaminieren können beispielsweise Kalandermaschinen verwendet werden. Das Aufbringen der Deckschicht erfolgt hierbei vorteilhafterweise unter leichtem Druck, um das Anhaften der Deckschicht an dem Schaum zu verbessern.

Allgemein kann festgestellt werden, dass eine befriedigende Trocknungsgeschwindigkeit der Schäume bereits bei 20 °C beobachtet wird, so dass eine Trocknung auf verletztem menschlichen oder tierischen Gewebe problemlos möglich ist. Für eine schnellere Trocknung und Fixierung der Schäume sollten jedoch Temperaturen oberhalb von 30 °C benutzt werden. Günstig sind Temperaturen von ≥ 80 °C bis ≤ 160 °C, vorzugsweise von ≥ 100 °C bis ≤ 150 °C, mehr bevorzugt von ≥ 120 °C bis ≤ 140 °C. Bei der Trocknung sollten jedoch Temperaturen von 200 °C nicht überschritten werden, da es anderenfalls zu unerwünschter Vergilbung der Schäume kommen kann. Möglich ist auch eine zwei- oder mehrstufige Trocknung.

Die Trocknung erfolgt in der Regel unter Verwendung von an sich bekannten Heiz- und Trockenapparaten, wie (Umluft-) Trockenschränken, Heißluft oder IR-Strahlern. Auch die Trocknung durch Führen des beschichteten Substrates über geheizte Oberflächen, zum Beispiel Walzen, ist möglich.

Der Auftrag sowie die Trocknung können jeweils diskontinuierlich oder kontinuierlich durchgeführt werden, bevorzugt ist jedoch ein gänzlich kontinuierliches Verfahren.

Die Polyurethan-Schäume können vor ihrer Trocknung typischerweise Schaumdichten von ≥ 50 g/Liter bis ≤ 800 g/Liter, bevorzugt ≥ 100 g/Liter bis ≤ 500 g/Liter, besonders bevorzugt ≥ 100 g/Liter bis ≤ 350 g/Liter (Masse aller Einsatzstoffe [in g] bezogen auf das Schaumvolumen von einem Liter).

Die Polyurethan-Schäume können nach ihrer Trocknung eine mikroporöse, zumindest teilweise offenporige Struktur mit miteinander kommunizierenden Zellen aufweisen. Die Dichte der getrockneten Schäume liegt dabei typischerweise unterhalb von 0,4 g/cm³, bevorzugt ist sie geringer als 0,35 g/cm³, besonders bevorzugt ≥ 0,01 g/cm³ bis ≤ 0,3 g/cm³ und liegt ganz besonders bevorzugt bei ≥ 0,1 g/cm³ bis ≤ 0,3 g/cm³.

Die Polyurethan-Schaumschichten, also die Schaumschicht und/oder die Deckschicht, haben nach dem Trocknen typischerweise eine Dicke von ≥ 0,1 mm bis ≤ 50 mm, bevorzugt ≥ 0,5 mm bis ≤ 20 mm, besonders bevorzugt ≥ 1 bis ≤ 10 mm, ganz besonders bevorzugt ≥ 1,5 mm bis ≤ 5 mm.

Der erhaltene Schichtenverbund kann schließlich noch unter Druck und Erwärmung verformt werden, um für seinen späteren Einsatzzweck angepasst zu werden.

Gegenstand der Erfindung ist weiterhin die Verwendung eines Schichtenverbundes gemäß der vorliegenden Erfindung als Sportartikel, Textilartikel, Kosmetikartikel oder Wundauflage. Bevorzugt ist die Verwendung als Wundauflage. Vorteilhafterweise kann die Wundauflage so geformt sein, dass sie auf Körperteile aufgelegt werden kann. Ein Beispiel für ein Körperteil ist die Ferse, die Stirn, das Kinn, der Hals, der Beckenkamm oder das Gesäß. Weiterhin kann das Körperteil beispielsweise ein Gelenk sein. Hinsichtlich ihrer Größe ist die Wundauflage dem aufzunehmenden Körperteil wie der Ferse oder einem Gelenk angepasst, also beispielsweise einem Fingergelenk, einem Ellenbogengelenk, einem Kniegelenk oder einem Fußgelenk.

Die vorliegende Erfindung wird anhand der nachfolgenden Zeichnung weiter erläutert. Es zeigen:

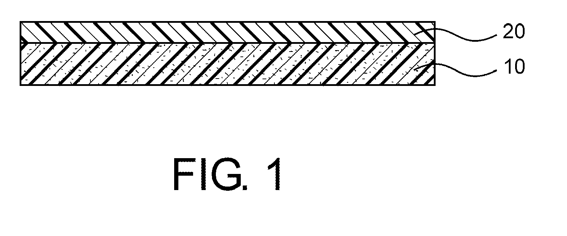

FIG 1 eine Querschnittsansicht eines erfindungsgemäßen Schichtenverbundes

FIG 1 zeigt eine Querschnittsansicht eines erfindungsgemäßen Schichtenverbundes. Die Schaumschicht 10 ist als Polyurethanschaumschicht ausgeführt, wobei der Polyurethanschaum wie beschrieben erhalten werden kann. Die Deckschicht 20, welche als thermoplastische Polyurethan-Membran ausgeführt ist, ist in direktem Kontakt mit der Schaumschicht 10. Bei der Verwendung des Schichtenverbundes wird die untere Oberfläche der Schaumschicht 10 auf die Wunde aufgelegt. Der poröse Schaum der Schaumschicht 10 kann überflüssiges Exsudat aus der Wunde aufnehmen. Wasserdampf kann durch die Deckschicht 20 hindurchdiffundieren, so dass ein der Wundheilung förderliches Klima in der Wunde aufgebaut werden kann. Gleichzeitig schützt die Deckschicht 20 die darunterliegende Schaumschicht 10 vor Verschmutzung und Keimbesiedlung. Der Schichtenverbund kann anschließend noch verformt werden, um ein an eine Körperkontur angepasste Wundauflage zu erhalten.

Die vorliegende Erfindung wird weiter anhand der nachfolgenden Beispiele erläutert.

Sofern nicht abweichend gekennzeichnet, beziehen sich alle Prozentangaben auf Gewichts-%.

Die Bestimmung der Festkörpergehalte erfolgte nach DIN-EN ISO 3251. NCO-Gehalte wurden, wenn nicht ausdrücklich anders erwähnt, volumetrisch gemäß DIN-EN ISO 11909 bestimmt. Die Bestimmung der "freien Saugleistung" erfolgte durch Absorption physiologischer Salzlösung nach DIN EN 13726-1, Teil 3.2.

Verwendete Substanzen und Abkürzungen:
- Diaminosulfonat:: NH₂-CH₂CH₂-NH-CH₂CH₂-SO₃Na (45 %ig in Wasser)
- Desmophen^{®} C2200:: Polycarbonatpolyol, OH-Zahl 56 mg KOH/g, zahlenmittleres Molekulargewicht 2000 g/mol (Bayer MaterialScience AG, Leverkusen, DE)
- PolyTHF^{®} 2000:: Polytetramethylenglykolpolyol, OH-Zahl 56 mg KOH/g, zahlenmittleres Molekulargewicht 2000 g/mol (BASF AG, Ludwigshafen, DE)
- PolyTHF^{®} 1000:: Polytetramethylenglykolpolyol, OH-Zahl 112 mg KOH/g, zahlenmittleres Molekulargewicht 1000 g/mol (BASF AG, Ludwigshafen, DE)
- Polyether LB 25:: monofunktioneller Polyether auf Ethylenoxid-/Propylenoxidbasis, zahlen- mittleres Molekulargewicht 2250 g/mol, OH-Zahl 25 mg KOH/g (Bayer MaterialScience AG, Leverkusen, DE)
- Pluronic^{®} PE 6800:: EO/PO-Blockcopolymer, gewichtsmittleres Molekulargewicht 8000 g/mol (BASF AG, Ludwigshafen, DE)
- Folie 1:: Polyether-Polyurethan-Membranfolie, Wasserdampfdurchlässigkeit (DIN 53122; 38 °C/90% rel. Feuchte): 4500 g/(m² × d)
- Folie 2:: Polyether-Polyurethan-Membranfolie, Wasserdampfdurchlässigkeit (DIN 53122; 38 °C/90% rel. Feuchte): 3000 g/(m² × d)
- Folie 3:: Polyether-Polyurethan-Membranfolie, Wasserdampfdurchlässigkeit (DIN 53122; 38 °C/90% rel. Feuchte): 2000 g/(m² × d)

Die Bestimmung der mittleren Teilchengrößen (angegeben ist das Zahlenmittel) der Polyurethan-Dispersion 1 erfolgte mittels Laserkorrelations-Spektroskopie (LKS; Gerät: Malvern Zetasizer 1000, Malver Inst. Limited).

Die für die Schaumadditive angegebenen Gehalte beziehen sich auf wässrige Lösungen.

### Beispiel 1: Herstellung der Polyurethan-Dispersion 1

1077,2 g PolyTHF^{®} 2000, 409,7 g PolyTHF^{®} 1000, 830,9 g Desmophen^{®} C2200 und 48,3 g Polyether LB 25 wurden in einer Standard-Rührapparatur auf 70 °C aufgeheizt. Anschließend wurde bei 70 °C innerhalb von 5 min ein Gemisch aus 258,7 g Hexamethylendiisocyanat und 341,9 g Isophorondiisocyanat zugegeben und bei 120 °C gerührt, bis der theoretische NCO-Wert erreicht oder leicht unterschritten war. Das fertige Prepolymer wurde mit 4840 g Aceton gelöst und dabei auf 50 °C abgekühlt und anschließend wurde eine Lösung aus 27,4 g Ethylendiamin, 127,1 g Isophorondiamin, 67,3 g Diaminosulfonat und 1200 g Wasser innerhalb von 10 min zudosiert. Die Nachrührzeit betrug 10 min. Dann wurde durch Zugabe von 654 g Wasser dispergiert. Es folgte die Entfernung des Lösemittels durch Destillation im Vakuum.

Die erhaltene Polyurethan-Dispersion hatte nachfolgende Eigenschaften:

| | |
|---|---|
| Feststoffgehalt: | 61,6 % |
| Partikelgröße (LKS): | 528 nm |
| pH (23°C): | 7,5 |

### Beispiele 2-6: Herstellung von Folien-Schaum-Laminaten aus der Polyurethan-Dispersion 1

120 g der Polyurethan-Dispersion 1, hergestellt nach Beispiel 1, wurden mit 12,6 g einer 30%igen Lösung von Pluronic^{®} PE 6800 in Wasser vermischt und unter Verwendung eines handelsüblichen Handrührgerätes (Rührer aus gebogenem Draht) auf 0,4 Liter Schaumvolumen aufgeschlagen. Mittels eines Filmziehgerätes (Rakel) mit Spalthöhe 6 mm wurde der Schaum auf nicht-haftendes Papier aufgezogen und für 20 Minuten bei 120 °C im Umluftrockenschrank getrocknet. Anschließend wurde eine Folie aus thermoplastischem Polyurethan (TPU-Folie; siehe Tabelle) auf den getrockneten Schaum aufgelegt und durch leichtes Wölben des 2-Schichtmaterials auf der konvexen Schaumseite falten- und blasenfrei auf dem Schaum fixiert. Nun wurde das Kompositmaterial für 30 Minuten bei der in der Tabelle angegebenen Temperatur im Trockenschrank getempert. Es wurden reinweiße Folien-Schaum-Laminate mit guten mechanischen Eigenschaften und feiner Porenstruktur erhalten.

| # | TPU-Folie | Foliendicke [µm] | Temperung [°C] | MVTR [g/24h/m²] | TFHC [g/24h/10cm²] | Schälfestigkeit [N/mm] |
|---|---|---|---|---|---|---|
| 2 | Folie 1 | 15 | 120 | 1840 | 15,9 | 0,03 |
| 3 | Folie 1 | 15 | 140 | 1650 | n.b. | 0,11 |
| 4 | Folie 2 | 15 | 120 | 1575 | 15,0 | n.b. |
| 5 | Folie 3 | 25 | 120 | 1137 | 9,3 | n.b. |
| 6 | Folie 3 | 30 | 120 | 1037 | 9,1 | n.b. |

| | | | | | | |
|---|---|---|---|---|---|---|
| n.b.: Wert wurde nicht bestimmt. MVTR: Feuchtigkeitstransportrate MVTR von Wundauflagen nach DIN EN 13726-2, Teil 3.2. TFHC: Feuchtigkeitsaufnahme und Rückhaltevermögen TFHC einer Wundauflage bei Kontakt mit Flüssigkeit nach DIN EN 13726-1, Teil 3.3. Schälfestigkeit: Zur Bestimmung wurden an einer Zwick Universalprüfmaschine 360° Peel-Prüfungen mit 100 mm/min Traversengeschwindigkeit durchgeführt. | | | | | | |

Die Beispiele zeigen, dass Schichtenverbunde, welche aufgrund ihrer Feuchtigkeitstransportrate und ihrer Feuchtigkeitsaufnahme als Wundauflage geeignet sind, mit Membranfolien laminiert werden können.

## Patentansprüche

1. Schichtenverbund, geeignet als Wundauflage, umfassend eine Schaumschicht (10) sowie eine Deckschicht (20), wobei die Deckschicht (20) ein thermoplastisches Polymer umfasst und zumindest teilweise direkt mit der Schaumschicht (10) verbunden ist und wobei die Schaumschicht (10) einen Polyurethanschaum umfasst, der erhalten wird, indem eine Zusammensetzung umfassend eine wässrige, anionisch hydrophilierte Polyurethandispersion (I) aufgeschäumt und getrocknet wird.

2. Schichtenverbund gemäß Anspruch 1, wobei die Zusammensetzung, aus der der Polyurethanschaum der Schaumschicht (10) erhalten wird, weiterhin Zusatzstoffe umfasst, die ausgewählt sind aus der Gruppe umfassend Fettsäureamide, Sulfosuccinamide, Kohlenwasserstoffsulfonate, Kohlenwasserstoffsulfate, Fettsäuresalze, Alkylpolyglycoside und/oder Ethylenoxid/Propylenoxid-Blockcopolymere.

3. Schichtenverbund gemäß Anspruch 2, wobei wobei die Ethylenoxid/Propylenoxid-Blockcopolymere eine Struktur gemäß der allgemeinen Formel (1) aufweisen: 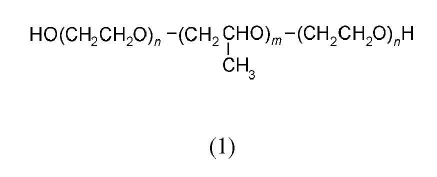 wobei der Wert für n in einem Bereich von ≥ 2 bis ≤ 200 liegt und der Wert für m in einem Bereich von ≥ 10 bis ≤ 60 liegt.

4. Schichtenverbund gemäß Anspruch 1, wobei die wässrige, anionisch hydrophilierte Polyurethandispersion (I) erhältlich ist, indem
A) isocyanatfunktionelle Prepolymere bereitgestellt werden, welche erhältlich sind aus einer Reaktionsmischung umfassend
A1) organische Polyisocyanate und
A2) polymere Polyole mit zahlenmittleren Molekulargewichten von ≥ 400 g/mol bis ≤ 8000 g/mol und OH-Funktionalitäten von ≥ 1,5 bis ≤ 6
und wobei anschließend
B) die freien NCO-Gruppen der Prepolymere ganz oder teilweise mit
B1) aminofunktionellen, anionischen oder potentiell anionischen Hydrophilierungsmitteln unter Kettenverlängerung umgesetzt werden und die Prepolymere vor, während oder nach Schritt B) in Wasser dispergiert werden, wobei weiterhin in der Reaktionsmischung vorliegende potentiell ionische Gruppen durch teilweise oder vollständige Umsetzung mit einem Neutralisationsmittel in die ionische Form überführt werden.

5. Schichtenverbund gemäß Anspruch 4, wobei die Reaktionsmischung in Schritt A) weiterhin umfasst:
A3) hydroxyfunktionelle Verbindungen mit Molekulargewichten von ≥ 62 g/mol bis ≤ 399 g/mol

6. Schichtenverbund gemäß Anspruch 4, wobei die Reaktionsmischung in Schritt A) weiterhin umfasst:
A4) isocyanatreaktive, anionische oder potentiell anionische und gegebenenfalls nichtionische Hydrophilierungsmittel

7. Schichtenverbund gemäß Anspruch 4, wobei in Schritt B) die freien NCO-Gruppen der Prepolymere weiterhin ganz oder teilweise umgesetzt werden mit
B2) aminofunktionelle Verbindungen mit Molekulargewichten von ≥ 32 g/mol bis ≤ 400 g/mol

8. Schichtenverbund gemäß Anspruch 4, wobei bei der Herstellung der wässrigen, anionisch hydrophilierten Polyurethan-Dispersionen (I) die Komponente A1) ausgewählt ist aus der Gruppe umfassend 1,6-Hexamethylendiisocyanat, Isophorondiisocyanat und/oder die isomeren Bis-(4,4'-isocyanatocyclohexyl)methane und wobei weiterhin die Komponente A2) eine Mischung aus Polycarbonatpolyolen und Polytetramethylenglykolpolyolen umfasst, wobei der Anteil der Summe der Polycarbonatpolyole und der Polytetramethylenglykolpolyetherpolyole an der Komponente A2) ≥ 70 Gewichts-% bis ≤ 100 Gewichts-% beträgt.

9. Schichtenverbund gemäß Anspruch 1, wobei das thermoplastische Polymer der Deckschicht (20) Materialien umfasst, die ausgewählt sind aus der Gruppe umfassend Polyurethan, Polyethylen, Polypropylen, Polyvinylchlorid, Polystyrol, Polyether, Polyester, Polyamid, Polycarbonat, Polyether-Polyamid-Copolymeren, Polyacrylat, Polymethacrylat und/oder Polymaleat.

10. Schichtenverbund gemäß Anspruch 1, wobei das thermoplastische Polymer der Deckschicht (20) Polyurethane umfasst, die ausgewählt sind aus der Gruppe umfassend aliphatische Polyesterpolyurethane, aromatische Polyesterpolyurethane, aliphatische Polyetherpolyurethane und/oder aromatische Polyetherpolyurethane.

11. Schichtenverbund gemäß Anspruch 1, wobei die direkte Verbindung zwischen der Schaumschicht (10) und der Deckschicht (20) eine Schälfestigkeit von 0,01 N/mm bis ≤ 0,50 N/mm aufweist.

12. Schichtenverbund gemäß Anspruch 1 mit einer Durchlässigkeit gegenüber Wasserdampf von ≥ 1000 g / 24 h × m² bis ≤ 4000 g / 24 h × m².

13. Verfahren zur Herstellung eines Schichtenverbundes gemäß Anspruch 1, umfassend die Schritte:
- Bereitstellen einer Schaumschicht, welche einen Polyurethanschaum umfasst, der erhalten wird, indem eine Zusammensetzung umfassend eine wässrige, anionisch hydrophilierte Polyurethandispersion (I) aufgeschäumt wird;
- Aufbringen einer Deckschicht (20) auf die Schaumschicht.

14. Verfahren gemäß Anspruch 13, wobei das Aufbringen der Deckschicht (20) auf die Schaumschicht derart ausgeführt wird, dass die Deckschicht (20) auf die zuvor getrocknete Schaumschicht auflaminiert wird.

15. Verwendung eines Schichtenverbundes gemäß Anspruch 1 als Sportartikel, Textilartikel, Kosmetikartikel oder Wundauflage.
